(19) Europäisches Patentamt
European Patent Office
Office européen des brevets

(11) **EP 4 473 990 A1**

(12) **EUROPEAN PATENT APPLICATION**

(43) Date of publication:
**11.12.2024 Bulletin 2024/50**

(51) International Patent Classification (IPC):
***A61M 5/32*** *(2006.01)*

(21) Application number: **23305890.8**

(52) Cooperative Patent Classification (CPC):
**A61M 5/3202;** A61M 2205/025

(22) Date of filing: **05.06.2023**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Becton Dickinson France
38800 Le Pont-de-Claix (FR)**

(72) Inventors:
• **BOSSHARDT, Michel
38640 Claix (FR)**
• **GIL, Ludovic
38000 Grenoble (FR)**
• **GIRARD, Benjamin
38320 Herbeys (FR)**

(74) Representative: **Germain Maureau
12, rue Boileau
69006 Lyon (FR)**

(54) **SYRINGE NEEDLE SHIELD FOR MINIMIZING NEEDLE CORROSION**

(57)     Provided herein is a needle shield for use with a prefilled or prefillable syringe. The syringe includes a syringe assembly having a barrel with a barrel end portion at the distal end thereof, a needle having a proximal end fixedly inserted into the barrel end portion, and a needle shield mounted to the barrel via the barrel end portion, so as to be positioned over the needle. A plunger is received within a chamber of the barrel and is axially movable therein. The needle shield includes a compliant material at least partially defining a hollow cavity therein, the hollow cavity including a needle receiving portion within which the needle is positioned when the needle shield is mounted to the barrel, with a wall forming part of the needle receiving portion having an angle, α, between 8° and 90° relative to a longitudinal axis of the needle shield.

FIG. 4

**Description**

**BACKGROUND OF THE INVENTION**

Field of the Invention

The present disclosure relates generally to syringes and, more particularly, to a needle shield for a syringe, with the needle shield configured to minimize corrosion of the needle of the syringe.

Description of Related Art

Syringes are used in a variety of environments for administering liquids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle and to preserve its sterility prior to use of the injection device.

Needle shields are generally formed to include a rigid component and a soft component. The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with an easily grippable surface for the user to remove the needle shield from the syringe.

While existing needle shields and the soft component therein provide adequate protection to the needle, it is recognized that existing needle shield designs can contribute to corrosion of the needle. That is, while the needle used for a prefilled syringe is typically made of stainless steel or another corrosion-resistant material, the needle may still be subject to corrosion under certain conditions - such as a combination of a humid environment, local confinement of the needle (such as within the needle shield), and contact with aggressive ions, e.g., chloride. As the elastic rubber used to form the soft component of the needle shield often includes chloride therein (due either to inorganic fillers therein that can bring traces of chloride and/or resulting from specific manufacturing steps of the pliant component), it is possible for chloride or other chemical species to leach from the rubber. This chloride may agglomerate on the needle if the needle wipes the rubber surface during assembly of the needle shield on the syringe, thus leading to an increased risk of corrosion development on the needle.

Accordingly, a need exists in the art for a needle shield that reduces the risk of corrosion developing on the needle.

**SUMMARY OF THE INVENTION**

Provided herein is a prefilled or prefillable syringe. The syringe includes a syringe assembly comprising a barrel having a barrel distal end and a barrel proximal end and defining a chamber, the barrel including a barrel end portion at the barrel distal end, a needle having a needle tip at a distal end of the needle and having a proximal end fixedly inserted into the barrel end portion, and a needle shield mounted to the barrel via the barrel end portion, so as to be positioned over the needle. The syringe also includes a plunger received within the chamber of the barrel and axially movable therein, wherein the needle shield comprises a compliant material at least partially defining a hollow cavity therein, the hollow cavity including a needle receiving portion within which the needle is positioned when the needle shield is mounted to the barrel, and characterized in that a wall forming part of an inner surface of the needle receiving portion has an angle, α, between 8° and 90° relative to a longitudinal axis of the needle shield.

In accordance with some aspects of the disclosure, the angle, α, defined as:

$$\alpha = tan^{-1}\left(\frac{W - w}{2(D - d)}\right)$$

where $W$ is a width of the hollow cavity at an open proximal end of the needle receiving portion, $w$ is a width of a tip portion of the needle receiving portion, $D$ is a length of the needle receiving portion, and $d$ is a length of the small diameter tip portion.

In accordance with some aspects of the disclosure, the length $D$ is constrained according to:

$$L\text{-}l > D$$

where $L$ is a length of the needle between the needle tip and the open proximal end of the needle shield cavity, and $l$ is a length of a bevel of the needle at needle tip.

In accordance with some aspects of the disclosure, $w = 0.4$ mm and is configured to receive any of a 25G, 27G, or 29G needle therein.

In accordance with some aspects of the disclosure, the angle, $\alpha$, is preferably between 9° and 90°, and more preferably between 9.5° and 90°.

In accordance with some aspects of the disclosure, a scrapping or wiping between the needle and the wall forming part of the inner surface of the needle receiving portion is reduced as a value of the angle, $\alpha$, increases.

In accordance with some aspects of the disclosure, the needle receiving portion comprises the open proximal end and a distal end, with the wall of the needle receiving portion tapering inward as the needle receiving portion progresses from the open proximal end to the distal end.

In accordance with some aspects of the disclosure, the tip portion is positioned at the distal end of the needle receiving portion, with the tip portion having an inner diameter larger than an outer diameter of the needle.

In accordance with some aspects of the disclosure, the tip portion of the needle receiving portion has a constant inner diameter.

In accordance with some aspects of the disclosure, the tip portion of the needle receiving portion houses a distal needle tip when the needle shield is mounted to the barrel.

In accordance with some aspects of the disclosure, the hollow cavity includes a barrel end storage portion configured to receive the barrel end portion.

In accordance with some aspects of the disclosure, the compliant material comprises rubber.

In accordance with some aspects of the disclosure, the needle shield comprises a rigid outer surface positioned about the compliant material.

## BRIEF DESCRIPTION OF THE DRAWINGS

FIG. 1 is a front view of a prefilled syringe, according to an embodiment of the disclosure;
FIG. 2 is a cross-sectional view taken along line A-A of FIG. 1;
FIG. 3 is an enlarged front view of a needle shield used for the prefilled syringe of FIGS. 1 and 2;
FIG. 4 is a cross-sectional view taken along line B-B of FIG. 3, according to an embodiment of the disclosure; and
FIG. 5 illustrates the cross-sectional view of FIG. 4 with the needle shield coupled to a prefilled syringe and covering a needle.

## DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

Aspects and embodiments of the disclosure are directed to a prefilled or prefillable syringe and an associated needle shield configured to inhibit corrosion on the syringe needle.

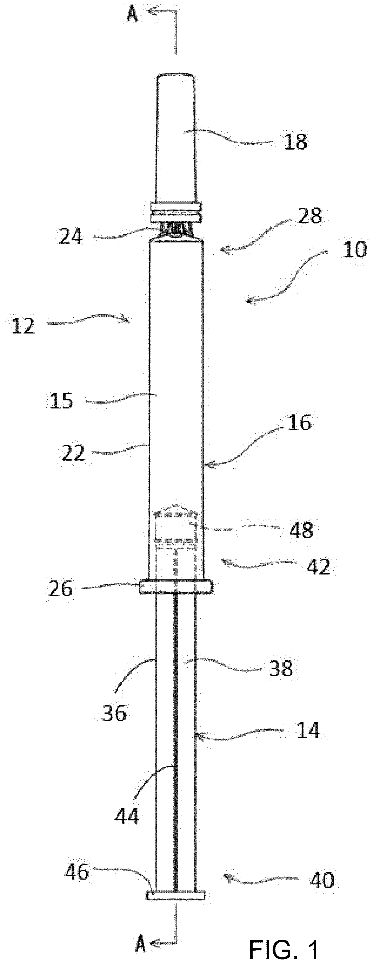

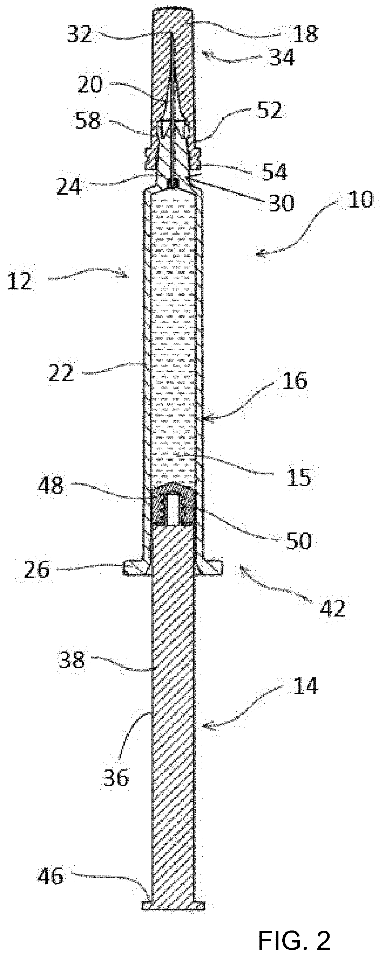

Referring to FIGS. 1 and 2, shown is a non-limiting embodiment of a syringe 10, such as a prefilled syringe for example, with which aspects or embodiments of the disclosure may be implemented. The prefilled syringe 10 includes a syringe assembly (i.e., a barrel with a puncture needle mounted with a cap) 12 and a plunger 14. The plunger 14 is stored in the syringe assembly 12 and slideable within the syringe assembly 12 in a liquid-tight manner. A medicament or other fluid is filled in a volume or chamber 15 formed by the syringe assembly 12 and the plunger 14.

According to aspects of the disclosure, the syringe assembly 12 includes a barrel 16, a needle shield 18 mounted to the barrel 16, and a needle 20 retained by the barrel 16. The barrel 16 includes a barrel body portion 22 that defines the chamber 15 within which the medicament or other fluid is contained, a cylindrical (hollow) barrel end portion 24 provided at the distal end of the barrel body portion 22, and a flange 26 provided at a proximal end of the barrel body portion 22. The barrel end portion 24 projects forward from a distal end 28 (shoulder portion) of the barrel body portion 22, and has a hollow cylindrical shape having a diameter smaller than that of the barrel body portion 22. A proximal end 30 of the needle 20 is fixedly inserted into the hollow portion of the barrel end portion 24 and may be fixed to the barrel end portion 24 with an adhesive, by thermal welding, or the like. The needle 20 is configured as a hollow needle (i.e., a lumen formed therein) that includes a needle tip 32 at a distal end 34 of the needle 20. The needle 20 may be formed of a suitable metal material, with stainless steel being an example. The hollow inside of the needle 20 communicates with the inner volume of the barrel 16.

As illustrated in FIGS. 1 and 2, the plunger 14 is formed of an elongate plunger rod 36 (more generally "plunger rod 36," as used hereafter) and a plunger head or stopper 48. The plunger rod 36 may include a main body 38 extending between a plunger proximal end 40 and a plunger distal end 42. In some embodiments, the main body 38 may include a plurality of elongate vanes or walls 44 extending axially along a length thereof between the plunger proximal end 40 and the plunger distal end 42. A thumb press 46 is positioned at the plunger proximal end 40 that may be engaged by a thumb (or other finger) of the user to apply a distally directed force to the plunger 14 to move the plunger rod 36 with respect to the barrel 16. A stopper 48 of plunger 14 is positioned at the plunger distal end 42 so as to be movable along with the plunger rod 36 within the chamber 15 of barrel 16. In some embodiments, one or more extension members 50 (e.g., disc-shaped flanges) are positioned at the plunger distal end 42 that are configured to mate with the stopper 48. In other embodiments, the plunger distal end 42 may include a female receptacle formed therein that is configured to receive and connect to a protrusion (e.g., pin) extending out proximally from the stopper 48, with the protrusion and receptacle engaging via threading, for example.

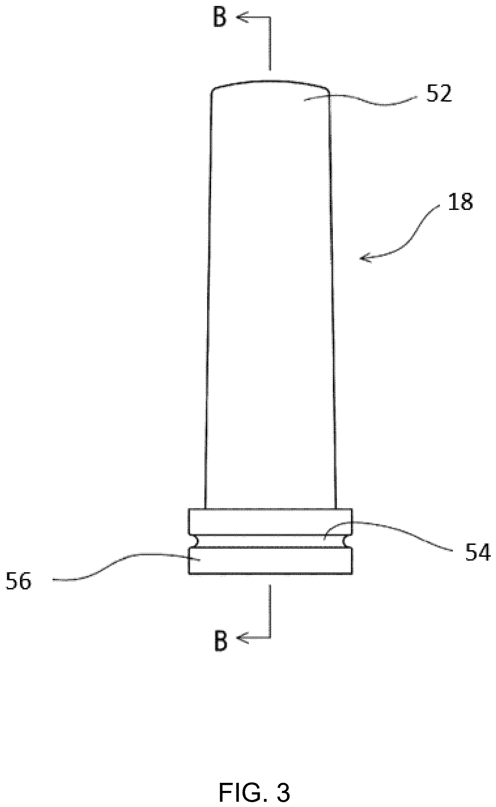

Referring still to FIGS. 1 and 2 and now also to FIGS. 3-5, according to aspects of the disclosure, the needle shield 18 is configured to be mounted to the barrel 16 via coupling of the needle shield 18 to the barrel end portion 24 of the barrel 16. The needle shield 18 may be structured such that at least a portion of an outer surface 52 thereof is formed of a rigid or semirigid material (e.g., a resin such as a polypropylene, a polyethylene, a polystyrene, a polyamide, a polycarbonate, or a polyvinyl chloride) that provides structure to the needle shield 18. In some embodiments, a flange 54 may be provided on the outer surface 52 at a proximal end 56 of the needle shield 18, with the flange 54 providing for gripping of the needle shield 18 to enable placement on the barrel 16 and/or removal from the barrel 16. The needle shield 18 also includes a compliant material 58 that forms an inner member 60 of the needle shield 18. The inner member 60 surrounds the needle 20 (when needle shield 18 is coupled to barrel 16) and may also present a mating surface for the barrel end portion 24. The compliant material 58 may be provided as rubber (e.g., butyl rubber, isoprene rubber, latex rubber, silicone rubber) or another elastomeric material.

According to aspects of the disclosure, the compliant material 58 of the needle shield 18 may be characterized as defining or including a closed distal end portion 62, an open proximal end portion 64, and a hollow cavity 66 that includes a barrel end storage portion 68 and a needle storage portion 70. The open proximal end portion 64 defines an opening that allows for positioning of the needle shield 18 onto the barrel end portion 24 of syringe assembly 12. The barrel end portion 24 may be introduced through open proximal end portion 64 and positioned within the barrel end storage portion 68, with the barrel end portion 24 sized to form a tight fit within the barrel end storage portion 68 to ensure engagement of the needle shield to the barrel 16.

The needle storage portion 70 is sized to receive the needle 20 therein, to provide protection to the needle 20 once the needle shield 18 is coupled to barrel 16. According to embodiments of the disclosure, the needle storage portion 70 has an inner diameter that tapers as the needle storage portion 70 extends from its proximal end 74 to its distal end 76. In some embodiments, the proximal end 74 of the needle storage portion 70 may be formed as a tapered portion having a diameter that reduces proximally to distally to prevent the insertion of the needle 20 into the compliant material 58 and securely guide the needle 20 distally. The distal end 76 of the needle storage portion 70 is formed with a small diameter tip portion 78 having an inner diameter, w, slightly larger than the outer diameter of the needle 20, with the tip portion 78 having a constant inner diameter.

In some embodiments, the hollow cavity 66 of needle shield 18 is configured such that, when the barrel end portion 24 of the barrel 16 is inserted into the needle shield 18 and is received in the barrel end storage portion 68 of the needle shield 18, the needle tip 32 of the needle 20 enters the small diameter tip portion 78 of the needle storage portion 70 but does not protrude therethrough so as to reach the compliant material 58 of closed distal end portion 62 that is positioned distally from the small diameter tip portion 78 of the needle storage portion 70 - i.e., the needle tip 32 does not puncture and extend into compliant material 58. Accordingly, contact between the needle tip 32 and the compliant material 58 is reduced or eliminated, and this reduction in contact between the needle tip 32 and the compliant material 58 may serve to prevent corrosion of the needle 20 that might occur responsive to chloride or other chemical species leaching from the compliant material 58 (i.e., rubber) and agglomerating on the needle 20 to cause corrosion thereof.

In accordance with other embodiments of the disclosure, a portion of the compliant material 58 distal from the needle storage portion 70 may be termed as a needle receiving portion 80 that allows for the insertion of the tip 32 of the needle 20 therein when the needle 20 is stored in the needle storage portion 70. When the needle shield 18 is mounted to the barrel end portion 24 of the barrel 16 and the barrel end storage portion 68 and the barrel 16 are engaged with each other, the needle may be stored with needle storage portion 70 and the needle tip 32 may protrude into and through small diameter tip portion 78 so as to be inserted and sealed in the needle receiving portion 80 of the needle shield 18.

According to aspects of the disclosure, the needle shield 18 is designed to minimize the risk of scrapping and/or wiping between the needle 20 and the inner surface of the needle shield 18 (i.e., inner surface of a portion of compliant material 58) during needle shield assembly, and to minimize the risk to create an undesired confinement zone/cavity between the needle shield 18 and the needle 20 in the area of the needle tip 32 during shelf-life. In order to achieve these results, the needle shield 18 is constructed to control dimensional ratios between the needle 20 and the needle shield 18.

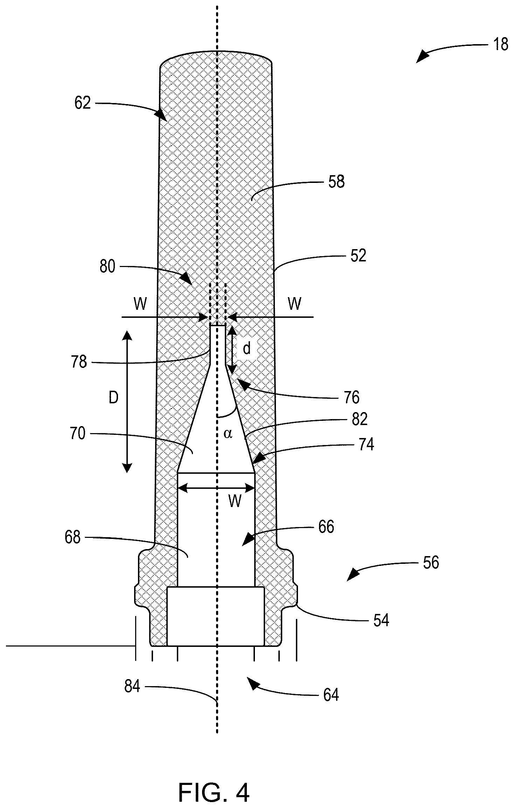

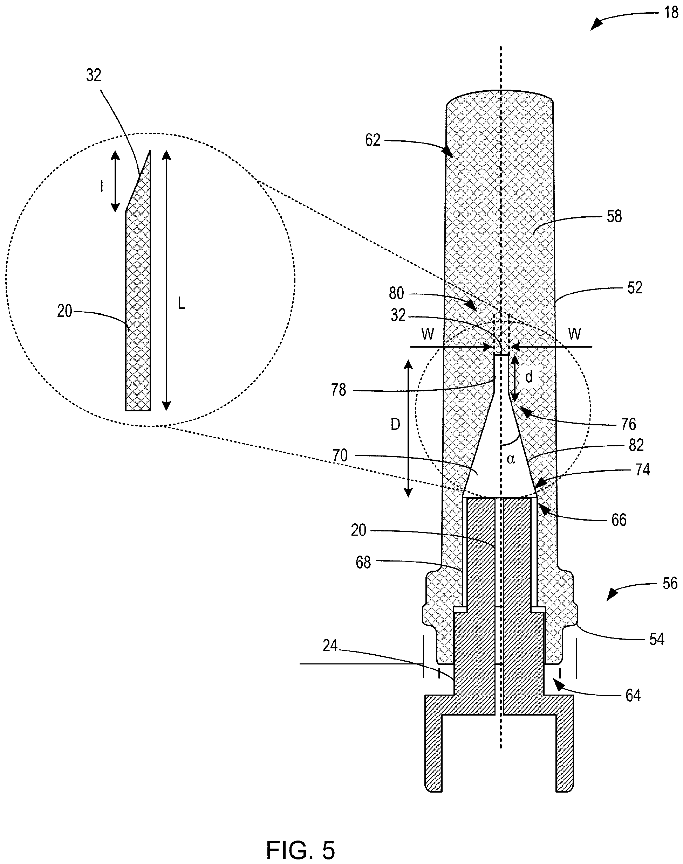

Referring now to FIGS. 4 and 5, features/dimensions of the hollow cavity 66 of needle shield 18 are shown that may be controlled to minimize the risk of scrapping/wiping between the needle 20 and the inner surface of the needle shield 18. The features/dimensions of the hollow cavity 66 that may be selectively controlled include: a width, W, of the hollow cavity 66 at an opening of needle storage portion 70 that is dependent on the outer diameter of the barrel end portion 24 (lower than the outer diameter of the barrel body portion 22); a width, w, of the small diameter tip portion 78; a length, D, of the needle storage portion 70; a length, d, of the small diameter tip portion 78; and an angle, alpha ($\alpha$), of an inner wall 82 of the compliant material 58 that forms the needle storage portion 70 of cavity 66 as the wall 82 tapers from the proximal end 74 of needle storage portion 70 to the distal end 76 of needle storage portion 70, with the angle, $\alpha$, measured from a longitudinal axis 84 of the needle shield 18. As indicated above, the dimensions of the features/dimensions of the needle storage portion 70 of needle shield 18 are controlled based on the dimensions of the needle 20, with the needle 20 defined by: a length, L, of the needle between the needle tip 32 and the basis of the needle storage portion 70; a length, l, of a bevel of the needle at needle tip 32; and an outer diameter or gauge of the needle 20. According to embodiments, needle shields 18 may be provided that are useable with needles of differing configurations, including needles of differing lengths and gauges, including 1/2" (12.7mm) and 5/8" (15.875mm) length needles, and 25G, 27G and 29G needles, as examples.

According to aspects of the disclosure, scrapping/wiping between the needle 20 and the inner surface of the compliant material 58 (including wall 82) may be minimized when the needle 20 and the inner surface of the wall 82 are perpendicular- which is achieved via (1) increasing a value of the angle, $\alpha$, or (2) reducing of the distance between the proximal part of the needle shield 18 (at opening of needle storage portion 70) and the inner distal part of the shield (at distal end of small diameter tip portion 78) where the needle 20 is pricked.

According to embodiments, in order to minimize scrapping/wiping between the needle 20 and the inner surface of the wall 82 while maintaining the sterility of the proximal part of the needle 20 during storage/transport, the needle shield 18 is configured to have an increased angle, $\alpha$. In optimizing the angle, $\alpha$, to minimize scrapping/wiping between the needle 20 and the inner surface of the wall 82, $\alpha$ may have a maximum value of 90° (where scrapping is minimized) and a minimum value of 8°, more preferably 9°, and even more preferably 9.5° (where scrapping is increased). The angle, $\alpha$, may be defined according to:

$$\alpha = tan^{-1}\left(\frac{W - w}{2(D - d)}\right)$$

with the constraints that w is a fixed value (based on the diameter/gauge of the needle 20, such as w = 0.4mm for 25G, 27G and 29G needles) and L-l > D, so that needle 20 is pricked inside the needle shield 18.

Provided here below are examples of needle shield configurations that may be utilized with standard length/gauge needles. While needle shield configurations for 1/2" and 5/8" length needles with a gauge of 25G, 27G or 29G are provided as specific examples for purposes of better illustrating aspects of the disclosure, it is recognized that needle shield configurations may be provided for needles covering all kinds of parenteral injections, including hypodermic needles or various configurations, with such needles having various lengths and/or gauges. As described above, the angle, $\alpha$, may be controlled to reduce scrapping/wiping between the needle 20 and the inner surface of the compliant material 58 (i.e., of wall 82), while maintaining the sterility of the proximal part of the needle 20 during storage/transport. Table 1 provides three (3) exemplary needle shields, according to non-limiting examples of the disclosure.

**TABLE 1**

| Shield Design | 5/8" needle (25G) | 5/8" needle (27G) | 1/2" needle (25G) |
|---|---|---|---|
| $w$ (mm) | 0.4 | 0.4 | 0.4 |
| $W$ (mm) | 3.81 | 3.81 | 3.8 |
| $d$ (mm) | 2 | 1.6 | 1.6 |
| $D$ (mm) | 13.94 | 11.71 | 9.15 |
| $\alpha$ (rad) | 0.141838 | 0.167073 | 0.221472 |
| $\alpha$ (deg) | 8.126743 | 9.545013 | 12.68941 |

It is recognized that needle shields having differing configurations from those indicated above may be provided to accommodate needles of differing structures, including needles of different lengths and gauges. In such embodiments, the needle shield will have an angle, $\alpha$, that reduces scrapping/wiping between the needle and the inner surface of the shield, while maintaining the sterility of the proximal part of the needle during storage/transport.

Beneficially, embodiments of the invention thus provide a needle shield that is configured to reduce the risk of corrosion developing on the needle. The needle shield includes an internal cavity shaped and dimensioned to accommodate receiving of the needle therein, while reducing contact between the needle and a compliant material of the needle shield.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

**Claims**

1.    A prefilled or prefillable syringe, the syringe comprising:

    a syringe assembly including:

        a barrel having a barrel distal end and a barrel proximal end and defining a chamber, the barrel including a barrel end portion at the barrel distal end;
        a needle having a needle tip at a distal end of the needle and having a proximal end fixedly inserted into the barrel end portion; and
        a needle shield mounted to the barrel via the barrel end portion, so as to be positioned over the needle; and

    a plunger received within the chamber of the barrel and axially movable therein;
    wherein the needle shield comprises a compliant material at least partially defining a hollow cavity therein, the hollow cavity including a needle receiving portion within which the needle is positioned when the needle shield is mounted to the barrel,
    **characterized in that** a wall forming part of an inner surface of the needle receiving portion has an angle, $\alpha$, between 8° and 90° relative to a longitudinal axis of the needle shield.

2.    The syringe of claim 1, wherein the angle, $\alpha$, defined as:

$$\alpha = tan^{-1}\left(\frac{W - w}{2(D - d)}\right)$$

where $W$ is a width of the hollow cavity at an open proximal end of the needle receiving portion, $w$ is a width of a tip portion of the needle receiving portion, $D$ is a length of the needle receiving portion, and $d$ is a length of the small diameter tip portion.

3. The syringe of claim 2, wherein the length $D$ is constrained according to:

$$L\text{-}l > D$$

where $L$ is a length of the needle between the needle tip and the open proximal end of the needle shield cavity, and $l$ is a length of a bevel of the needle at needle tip.

4. The syringe of claim 2 or claim 3, wherein $w = 0.4$ mm and is configured to receive any of a 25G, 27G, or 29G needle therein.

5. The syringe of any of claims 1-4, wherein the angle, $\alpha$, is preferably between 9° and 90°, and more preferably between 9.5° and 90°.

6. The syringe of any of claims 1-5, wherein a scrapping or wiping between the needle and the wall forming part of the inner surface of the needle receiving portion is reduced as a value of the angle, $\alpha$, increases.

7. The syringe of any of claims 1-6, wherein the needle receiving portion comprises the open proximal end and a distal end, with the wall of the needle receiving portion tapering inward as the needle receiving portion progresses from the open proximal end to the distal end.

8. The syringe of claim 7, wherein the tip portion is positioned at the distal end of the needle receiving portion, with the tip portion having an inner diameter larger than an outer diameter of the needle.

9. The syringe of claim 8, wherein the tip portion of the needle receiving portion has a constant inner diameter.

10. The syringe of claim 8 or claim 9 wherein the tip portion of the needle receiving portion houses a distal needle tip when the needle shield is mounted to the barrel.

11. The syringe of any of claims 1-10, wherein the hollow cavity includes a barrel end storage portion configured to receive the barrel end portion.

12. The syringe of any of claims 1-11, wherein the compliant material comprises rubber.

13. The syringe of any of claims 1-12, wherein the needle shield comprises a rigid outer surface positioned about the compliant material.

FIG. 1

FIG. 2

B

52

18

56

54

B

FIG. 3

FIG. 4

FIG. 5

Europäisches
Patentamt

European
Patent Office

Office européen
des brevets

# EUROPEAN SEARCH REPORT

Application Number

EP 23 30 5890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/044608 A1 (ODELL ROBERT B [US] ET AL) 22 November 2001 (2001-11-22) * figures 1, 2, 3, 4, 5, 6, 7, 8, 9, 10, 11, 12 * * paragraphs [0021] – [0032] * | 1-13 | INV. A61M5/32 |
| X | EP 3 192 549 A1 (TERUMO CORP [JP]) 19 July 2017 (2017-07-19) * figures 1, 2, 3, 4, 5, 6, 7, 8, 9, 10, 11, 12 * * column 4, line 64 – column 9, line 59 * | 1-13 | |
| A | US 4 986 818 A (IMBERT CLAUDE [FR] ET AL) 22 January 1991 (1991-01-22) * the whole document * | 1-13 | |
| A | US 2018/344942 A1 (SWAL MICKAËL [FR] ET AL) 6 December 2018 (2018-12-06) * the whole document * | 1-13 | |
| A | US 2008/215013 A1 (FELIX-FAURE CATHERINE [FR]) 4 September 2008 (2008-09-04) * the whole document * | 1-13 | TECHNICAL FIELDS SEARCHED (IPC) A61M |

The present search report has been drawn up for all claims

| Place of search | Date of completion of the search | Examiner |
|---|---|---|
| Munich | 20 October 2023 | Benes, Václav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5890

This annex lists the patent family members relating to the patent documents cited in the above-mentioned European search report.
The members are as contained in the European Patent Office EDP file on
The European Patent Office is in no way liable for these particulars which are merely given for the purpose of information.

20-10-2023

| Patent document cited in search report | | Publication date | Patent family member(s) | | Publication date |
|---|---|---|---|---|---|
| US 2001044608 | A1 | 22-11-2001 | DE | 69925205 T2 | 09-03-2006 |
| | | | EP | 0976415 A2 | 02-02-2000 |
| | | | JP | 2000051354 A | 22-02-2000 |
| | | | US | 2001044608 A1 | 22-11-2001 |
| EP 3192549 | A1 | 19-07-2017 | EP | 3192549 A1 | 19-07-2017 |
| | | | JP | 6686268 B2 | 22-04-2020 |
| | | | JP | WO2016039111 A1 | 29-06-2017 |
| | | | US | 2017182259 A1 | 29-06-2017 |
| | | | WO | 2016039111 A1 | 17-03-2016 |
| US 4986818 | A | 22-01-1991 | NONE | | |
| US 2018344942 | A1 | 06-12-2018 | CN | 108136135 A | 08-06-2018 |
| | | | DK | 3352821 T3 | 04-11-2019 |
| | | | EP | 3352821 A1 | 01-08-2018 |
| | | | FR | 3041263 A1 | 24-03-2017 |
| | | | IL | 257906 A | 31-05-2018 |
| | | | JP | 6926069 B2 | 25-08-2021 |
| | | | JP | 2018528014 A | 27-09-2018 |
| | | | KR | 20180058761 A | 01-06-2018 |
| | | | PL | 3352821 T3 | 31-03-2020 |
| | | | TW | 201711718 A | 01-04-2017 |
| | | | US | 2018344942 A1 | 06-12-2018 |
| | | | WO | 2017051108 A1 | 30-03-2017 |
| US 2008215013 | A1 | 04-09-2008 | CN | 101301502 A | 12-11-2008 |
| | | | EP | 1964588 A1 | 03-09-2008 |
| | | | FR | 2913202 A1 | 05-09-2008 |
| | | | JP | 2008229328 A | 02-10-2008 |
| | | | US | 2008215013 A1 | 04-09-2008 |

EPO FORM P0459

For more details about this annex : see Official Journal of the European Patent Office, No. 12/82